Europäisches Patentamt

European Patent Office

Office européen des brevets

(19)

(11) Veröffentlichungsnummer: **0 163 799 B1**

(12) **EUROPÄISCHE PATENTSCHRIFT**

(45) Veröffentlichungstag der Patentschrift: 18.09.91

(21) Anmeldenummer: 84730094.4

(22) Anmeldetag: 04.09.84

(51) Int. Cl.⁵: **G01N 33/543**, G01N 33/548

(54) Mit Antigenen oder Antikörpern beschichteter Träger.

(30) Priorität: 04.05.84 DE 3416933

(43) Veröffentlichungstag der Anmeldung:
11.12.85 Patentblatt 85/50

(45) Bekanntmachung des Hinweises auf die
Patenterteilung:
18.09.91 Patentblatt 91/38

(84) Benannte Vertragsstaaten:
AT BE CH DE FR GB IT LI LU NL SE

(56) Entgegenhaltungen:
EP-A- 0 063 810
DE-B- 2 734 118
US-A- 4 094 647

analyt.Chem. 131,1-15(1983)

Analyt.Biochem.124,396-405(1982)

(73) Patentinhaber: Köhler, Dora
Kreutzer Weg 11
W-1000 Berlin 45(DE)

(72) Erfinder: Köhler, Dora
Kreutzer Weg 11
W-1000 Berlin 45(DE)

(74) Vertreter: Pfenning, Meinig & Partner
Kurfürstendamm 170
W-1000 Berlin 15(DE)

## Beschreibung

Die Erfindung betrifft einen mit Antigenen oder Antikörpern beschichteten Träger, der mit einem in einem Gefäß aufgenommenen Serum oder Körpersaft von Mensch, Tier oder Pflanze eine serologische Reaktion durchführt.

Aus dem Stand der Technik sind Gefäße zur Durchführung serologischer Reaktionen bekannt, die als Mikrotiter bezeichnet werden und die aus einer Vielzahl von in einer Kunststoffkarte angeordneten nebeneinanderliegenden Vertiefungen bestehen. Derartige Mikrotiter werden in einem aufwendigen Verfahren mit besonders aufbereiteten Antigenen oder Antikörpern beschichtet. Das zu untersuchende Serum wird in die Vertiefungen des Gefäßes eingebracht und die in dem Serum vorhandenen Antikörper binden sich mit den Antigenen. In dem weiteren Reaktionsvorgang kann diese Bindung erkennbar gemacht werden. Dieses bekannte Verfahren, bei dem das Gefäß gleichzeitig den mit den Antigenen bzw. Antikörpern beschichteten Träger darstellt, weist mehrere Nachteile auf. Während des stufenweisen serologischen Reaktionsvorganges, insbesondere durch die zwischengeschaltete Spülung, löst sich teilweise die Bindung der Antigene und der Antikörper wieder, so daß zwischen 30 % und 70 % der ursprünglich gebundenen Antigene und Antikörper verloren gehen. Daher sind standardisierte und reproduzierbare Untersuchungsergebnisse nicht oder nur begrenzt möglich. Dadurch, daß die Träger als Gefäße ausgebildet sind und daß die nebeneinanderliegenden Vertiefungen des Mikrotiters jeweils nur mit einer Sorte von Antigenen beschichtet werden können, ist die allgemeine Anwendung für die serologische Früh-, Schnell- und Differentialdiagnose von Krankheiten und deren Erregern nur schwer möglich. Die Herstellung der Träger in Gefäßform ist wegen des hohen materiellen und technischen Aufwandes äußerst kostenintensiv, so daß auch von daher die Anwendungsmöglichkeiten der Träger nach dem Stand der Technik aufgrund der hohen Kosten zusätzlich eingeschränkt sind.

Hier setzt die vorliegende Erfindung ein, der die Aufgabe zugrunde liegt, einen mit Antigenen bzw. Antikörpern beschichteten Träger für serologische Reaktionen in Zusammenhang mit in der Serologie verwendeten Gefäßen zu schaffen, der mit einem geringen technischen und materiellen Aufwand herstellbar und einfach anzuwenden ist, wobei die Untersuchungsergebnisse standardisierbar und reproduzierbar sein sollen und eine Früh-, Schnell- und Differentialdiagnose von Krankheiten und Krankheitserregern erlauben.

Diese Aufgabe wird erfindungsgemäß durch ein Verfahren zur Bestimmung von mittels Antigenen und Antikörpern zu untersuchenden Proben mit einer Mikrotiterplatte mit Ausnehmungen zur Aufnahme der Proben und mit einem Träger, der Plättchen oder Streifen, an einer Halteleiste befestigt, aufnimmt, wobei die Streifen in die Ausnehmungen der Mikrotiterplatte eingesetzt werden, dadurch gekennzeichnet, daß als Streifen solche aus Nitrozellulose verwendet werden, wobei die Durchführung serologischer Reaktionen (Massenuntersuchungen) dadurch erfolgt, daß die Nitrozellulose von einem Halteelement aus Kunststoff aufgenommen und auf einer Seite mit einem Kunststoffstreifen verstärkt wird und mehrere Nitrozellulosestreifen im Abstand nebeneinander kammförmig angeordnet und so von den Ausnehmungen in der Mikrotiterplatte aufgenommen werden.

Dadurch, daß als Träger Nitrozellulose verwendet wird, die mit den Antigenen bzw. Antikörpern beschichtet ist, und dadurch, daß die Nitrozellulose in ihrer Form an die in der Serologie Üblichen Gefäße durch einfaches Zerschneiden oder dergleichen anpaßbar ist, wird eine breite Anwendung und eine verbesserte Praktikabilität durch geringeren Aufwand bei Ärzten, in Kliniken und Laboratorien ermöglicht. Durch die Verwendung der erfindungsgemäßen Träger ist eine einfache Differentialdiagnose möglich, dabei beispielsweise bei einem Mikrotiter mit der Vielzahl von das Serum bzw. Körpersäften aufnehmenden Vertiefungen Träger mit unterschiedlichen Antigenen bzw. Antikörpern anwendbar sind.

Die erfindungsgemäßen Träger binden Antigene und Antikörper unterschiedlicher Herkunft ohne aufwendige Aufbereitung vollständig und dauerhaft und es treten auch während der stufenartigen serologischen Reaktion keine Antigen- und Antikörperverluste auf, so daß standardisierbare und reproduzierbare Untersuchungsergebnisse möglich werden.

Die Träger sind mit geringem technischen und materiellen Aufwand herstellbar, so daß die Herstellungskosten im Vergleich zu den Trägern nach dem Stand der Technik sehr viel geringer sind. Die mit Antigenen bzw. Antikörpern beschichteten Träger können ohne den Verlust ihrer Aktivität langfristig aufbewahrt werden.

Durch die in den Unteransprüchen angegebenen Maßnahmen sind vorteilhafte Weiterbildungen und Verbesserungen möglich.

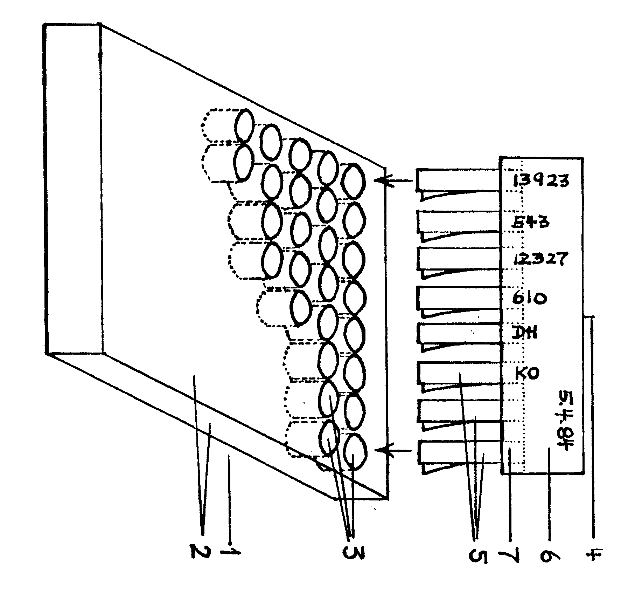

Die Erfindung ist in der Zeichnung dargestellt und wird in der nachfolgenden Beschreibung näher erläutert.

Es zeigen die einzige Figur ein in der Serologie angewandtes, als Mikrotiter bezeichnetes Gefäß und einen mit diesem Gefäß verwendeten Träger in perspektivischer Ansicht.

In der Figur ist ein in der Serologie verwendetes Gefäß 1, das als Mikrotiter bezeichnet wird, dargestellt. Das Gefäß 1 ist derart ausgebildet, daß in einem Rahmen 2 aus Kunststoff ein Vielzahl von Vertiefungen 3 vorgesehen sind, die matrixartig angeordnet sind. In den Vertiefungen 3 befindet sich das zu untersuchende Material aus Menschen, Tieren oder Pflanzen, das Serum oder Körpersäfte, wie Urin, Liquor, Blut u.s.w,, sein kann.

Der Träger 4 ist kammartig ausgebildet und besteht aus mehreren Streifen 5, die in einem Halteelement 6 gehalten werden. Die Streifen 5 bestehen aus Nitrozellulose, einem anderen Material, das der Nitrozellulose entsprechende Adsorptionseigenschaften besitzt oder aus strukturellen und chemischen Analogen, und sind mit Antigenen bzw. Antikörpern beschichtet, wobei diese Beschichtung nach dem Zuschnitt der Nitrozellulose in die jeweilige Form, in dem dargestellten Ausführungsbeispiel in die Streifen, erfolgt. Das Halteelement 6 besteht beispielsweise aus einer Kunststoffolie, auf der die Streifen 5 mittels Klebestreifen 7 im Abstand zueinander festgelegt sind. Das Halteelement 6 kann mit Daten bezüglich des zu untersuchenden Materials usw. beschriftet werden. Zur Verstärkung und zum Schutz der Streifen 5 gegen Beschädigungen können der Form der Streifen 5 entsprechende Streifen aus Kunststoff vorgesehen sein, die einseitig als Auflage dienend, mit den Streifen 5 in dem Halteelement 6 aufgenommen sind. Je nach Ausführung sind die Streifen 5 mit gleichen oder unterschiedlichen Arten von Antigenen bzw. Antikörpern beschichtet.

Die jeweils vorbehandelten Träger werden in die Vertiefungen 3 verbracht, in denen sich das zu untersuchende Material aus Menschen, Tieren oder Pflanzen befindet. Die serologische Reaktion findet auf dem Träger statt, auf dem sich die in dem zu untersuchenden Material vorhandenen Antikörper bzw. Antigenen mit den Antigenen bzw. Antikörpern binden. Die Streifen werden mit einem Reagenz, z.B. Fastred in Verbindung mit Naphtol, behandelt, das sich als Folge der serologischen Reaktionen verfärbt, wobei das Reaktionsergebnis durch diese Verfärbung des Trägers 4 optisch, photometrisch oder mikroskopisch erkennbar ist. Die Art der Anfärbung des Trägers 4 stellt das Untersuchungsergebnis dar.

In dem beschriebenen Ausführungsbeispiel ist das Gefäß 1 als Mikrotiter und der Träger 4 mit mehreren nebeneinanderliegenden Streifen 5 versehen. Selbstverständlich können auch andere Gefäße, beispielsweise solche mit einer Vertiefung und einzelne Streifen oder Nitrozellulose in anderer Form verwendet werden.

In einem anderen nicht dargestellten Ausführungsbeispiel sind die Träger als Rundstücke ausgebildet und in die Vertiefungen 3 eingelegt und mit diesen, beispielsweise durch Klebungen, verbunden. Zur Durchführung der serologischen Reaktionen wird das zu untersuchende Material in die Vertiefungen eingebracht und die serologische Reaktion findet, wie oben beschrieben, auf den einzelnen Trägern statt.

## Patentansprüche

1. Verfahren zur Bestimmung von mittels Antigenen und Antikörpern zu untersuchenden Proben mit einer Mikrotiterplatte mit Ausnehmungen zur Aufnahme der Proben und mit einem Träger, der Plättchen oder Streifen, an einer Halteleiste befestigt, aufnimmt, wobei die Streifen in die Ausnehmungen der Mikrotiterplatte eingesetzt werden, dadurch gekennzeichnet, daß als Streifen solche aus Nitrozellulose verwendet werden, wobei die Durchführung serologischer Reaktionen (Massenuntersuchungen) dadurch erfolgt, daß die Nitrozellulose von einem Halteelement aus Kunststoff aufgenommen und auf einer Seite mit einem Kunststoffstreifen verstärkt wird und mehrere Nitrozellulosestreifen im Abstand nebeneinander kammförmig angeordnet und so von den Ausnehmungen in der Mikrotiterplatte aufgenommen werden.

## Claims

1. Process for determining samples to be examined by means of antigens and antibodies, using a micro-titration plate with recesses for receiving samples and with a carrier which accommodates platelets or strips which are mounted to a holding ledge, in which respect the strips are entered into the recesses of the micro-tritration plate, **characterised in that** the strips are of nitro-cellulose, whereby seroligical reactions (mass examinations) are carried out in that the nitro-cellulose is received by a holding element of synthetic material and on one side reinforced with a synthetic-material strip and that several nitro-cellulose strips are comb-like arranged a distance apart alongside one another and thus accommodated by the recesses in the micro-titration plate.

## Revendications

1. Procédé de détermination d'échantillons à examiner au moyen d'antigènes ou d'anticorps avec une plaque de microtitrage munie de cavités pour le logement d'échantillons et d'un support fixé à un bord de maintien qui reçoit les lamelles ou les bandes, les bandes étant placées dans les cavités de la plaque de microtitrage, caractérisé en ce que l'on utilise comme bandes des bandes en nitrocellulose grâce auxquelles se produit la marche des réactions sérologiques (recherches de masse), en ce que la nitrocellulose est maintenue par un élément de maintien en matière plastique et renforcée sur un côté par une bande de matière plastique et que plusieurs bandes de nitrocellulose sont disposées en forme de peigne à une certaine distance l'une de l'autre et sont ainsi recueillies par les cavités de la plaque de microtitrage.